# EUROPEAN PATENT APPLICATION

(11) **EP 4 302 676 A1**
(43) Date of publication of application: **10.01.2024**
(21) Application number: 23163830.5
(22) Date of filing: 23.03.2023
(51) Int. Cl.: A61B 1/00, A61B 1/015, A61B 1/018

(54) **ENDOSCOPE COMPRISING A Y-CONNECTOR**

(30) Priority: 25.03.2022 JP 2022050774
(71) Applicant: FUJI-FILM Corporation, Minato-ku Tokyo 106-8620 (JP)
(72) Inventor: KUNUKI, Yoshiyuki, Kanagawa, 2588538 (JP)
(74) Representative: Kudlek, Franz Thomas

(57) **Abstract**

An endoscope includes: an insertion pipe line through which a treatment tool is inserted; a suction pipe line; an introduction pipe line into which the treatment tool is introduced; and a branching member that branches the insertion pipe line into the introduction pipe line and the suction pipe line, in which a proximal end portion of the insertion pipe line is inserted into the branching member, the proximal end portion includes a first part and a second part that is provided at a proximal end side with respect to the first part, and an opening facing an introduction pipe line side is formed in an outer peripheral surface of the second part.

## Description

### BACKGROUND OF THE INVENTION

### 1. Field of the Invention

The present invention relates to an endoscope.

### 2. Description of the Related Art

JP2002-95633A discloses an endoscope in which a branch passage forming body is inserted into a treatment tool insertion channel.

JP1987-281917A (JP-S62-281917A) discloses an endoscope in which a branch block and a channel pipe are connected to each other via a connecting pipe body.

### SUMMARY OF THE INVENTION

In JP2002-95633A, there is a probability that a treatment tool may be caught at a joint between the treatment tool insertion channel and the branch passage forming body. In JP1987-281917A (JP-S62-281917A), there is a probability that a treatment tool may be caught at a joint between the branch block and the connecting pipe body.

An object of the present invention is to provide an endoscope capable of smoothly taking in and out a treatment tool.

According to one embodiment of the technology of the present disclosure, there is provided an endoscope comprising: an insertion pipe line through which a treatment tool is inserted; a suction pipe line; an introduction pipe line into which the treatment tool is introduced; and a branching member that branches the insertion pipe line into the introduction pipe line and the suction pipe line, in which a proximal end portion of the insertion pipe line is inserted into the branching member, the proximal end portion includes a first part and a second part that is provided at a proximal end side with respect to the first part, and an opening facing an introduction pipe line side is formed in an outer peripheral surface of the second part.

According to the present invention, it is possible to provide an endoscope capable of smoothly taking in and out a treatment tool.

### BRIEF DESCRIPTION OF THE DRAWINGS

Fig. 1 is a schematic perspective view of an endoscope system 100 provided with an endoscope 1, which is an embodiment of the present invention.
Fig. 2 is an enlarged perspective view of an operating device 2 shown in Fig. 1 as viewed obliquely from a front side.
Fig. 3 is a perspective view of the operating device 2 shown in Fig. 2 as viewed obliquely from a rear side.
Fig. 4 is an exploded perspective view of the operating device 2.
Fig. 5 is an exploded perspective view of a suction unit 10.
Fig. 6 is an exploded perspective view showing a structure in which a suction nozzle 12 is incorporated into a holding member 5.
Fig. 7 is a cross-sectional perspective view showing a structure that restricts a rotational movement range of the suction nozzle 12.
Fig. 8 is a cross-sectional view of the suction unit 10 in an assembled state.
Fig. 9 is an enlarged cross-sectional view of the periphery of a branching member 50.
Fig. 10 is an exploded perspective view of each component shown in Fig. 9.
Fig. 11 is a perspective view showing a proximal end portion 20a of an insertion pipe line 20.
Fig. 12 is a partially enlarged cross-sectional perspective view of the branching member 50.
Figs. 13A and 13B are perspective views showing two modification examples of the proximal end portion 20a of the insertion pipe line 20.
Fig. 14 is an enlarged perspective view showing an attachment structure of a bending operation portion 6 and of a circuit board 7.
Fig. 15 is an exploded perspective view showing a state in which each component is incorporated into a first case member 3.
Fig. 16 is a cross-sectional view taken along line A-A of Fig. 15.
Fig. 17 is an enlarged perspective view of a lower part in Fig. 15.
Fig. 18 is a perspective view showing an inner surface of the first case member 3.
Fig. 19 is a perspective view showing an inner surface of a second case member 4.
Fig. 20 is an enlarged exploded perspective view of a lower end part of a housing 9.

### DESCRIPTION OF THE PREFERRED EMBODIMENTS

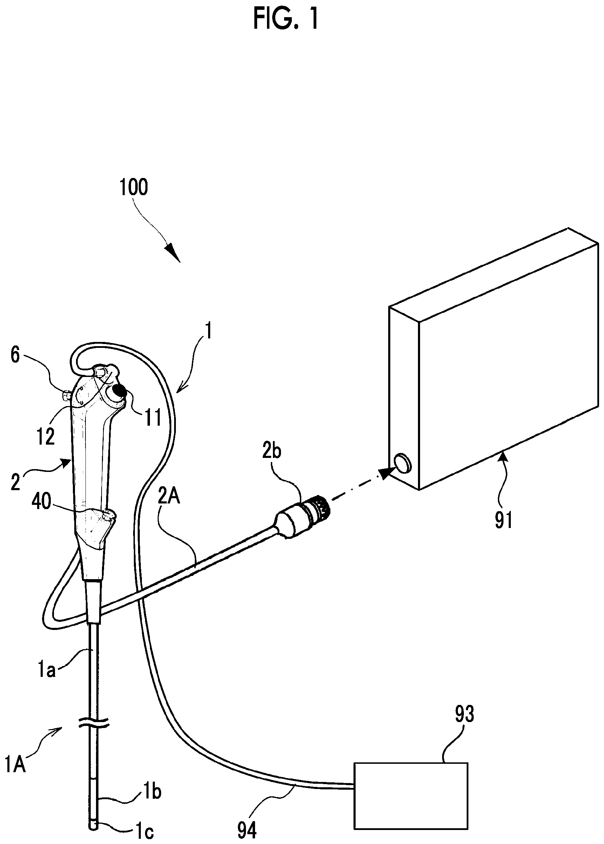

Fig. 1 is a schematic perspective view of an endoscope system 100 provided with an endoscope 1, which is an embodiment of the present invention.

The endoscope system 100 comprises the endoscope 1, a case 91 incorporating a tablet terminal and an interface adapter that connects the tablet terminal and the endoscope 1 to each other, and an external device, such as a suction device 93.

The endoscope 1 comprises an insertion part 1A that is a tubular member extending in one direction and that is inserted into a body cavity as an object to be observed, an operating device 2 that is consecutively provided at a proximal end part of the insertion part 1A and that is provided with an operating member for performing a forceps operation, a suction operation, and the like, and a cable 2A, such as a universal cord including a connector portion 2b, that attachably and detachably connects the endoscope 1 to the interface adapter of the case 91. The insertion part 1A is formed of a flexible tube or the like. The endoscope 1 is for single use, but the present invention is not limited thereto. It should be noted that the single-use endoscope is not strongly required to have liquid-tightness and durability in the operating device 2.

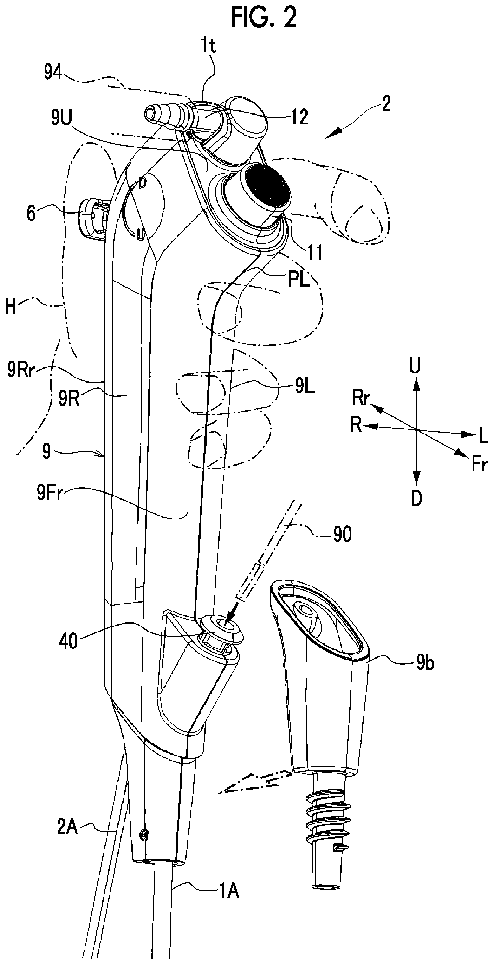

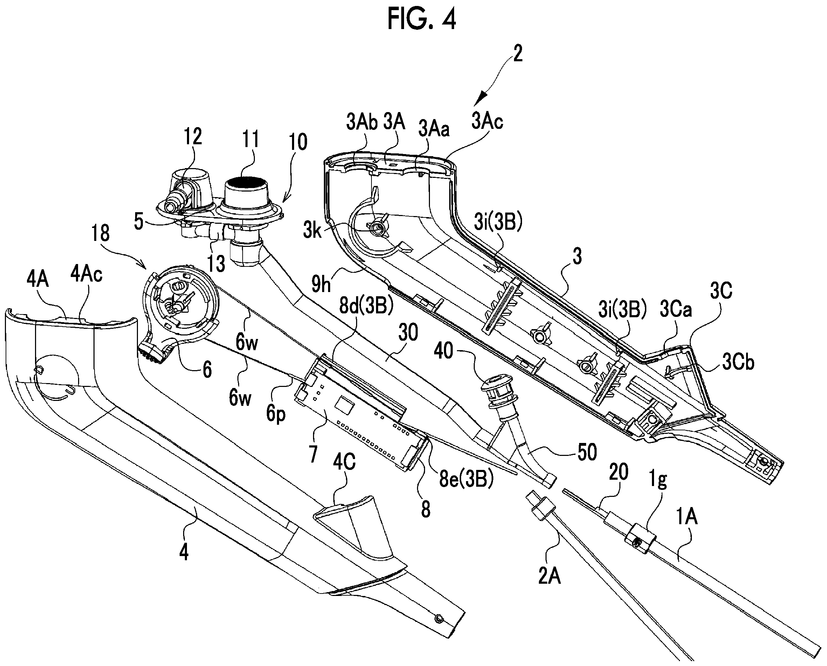

The operating device 2 is provided with an introduction pipe line 40 provided with an introduction opening for introducing a treatment tool 90 (see Fig. 2), such as a biopsy forceps or an electric scalpel, which is a collection device for collecting a biological tissue, such as a cell or a polyp, into an insertion pipe line 20 (see Fig. 4) provided inside the insertion part 1A. The insertion part 1A is composed of a flexible soft portion 1a, a bendable portion 1b provided at a distal end of the soft portion 1a, and a rigid distal end portion 1c provided at a distal end of the bendable portion 1b. The bendable portion 1b is bendable through a rotational movement operation of a bending operation portion 6 provided in the operating device 2. The bendable portion 1b can be bent in any direction at any angle depending on a part of the subject or the like for which the endoscope 1 is used, and the distal end portion 1c can be directed in a desired direction. The insertion pipe line 20 is provided so as to extend from a proximal end to a distal end of the insertion part 1A. The operating device 2 is provided with a suction button 11 and a suction nozzle 12 connected to the suction device 93 via a suction pipe 94.

In the following description, in the operating device 2, three orthogonal directions will be referred to as an up-down direction, a front-rear direction, and a left-right direction in descending order of length. The left-right direction corresponds to a thickness direction of a hand in a case in which the operating device 2 is gripped by the hand in a normal posture. The up-down direction is a longitudinal direction of the operating device 2. In the following description, for convenience, a front side, a rear side, a left side, a right side, an upper side, and a lower side are defined as illustrated in Fig. 2, and the front side is indicated by Fr, the rear side is indicated by Rr, the left side is indicated by L, the right side is indicated by R, the upper side is indicated by U, and the lower side is indicated by D. Regarding the up-down direction, the upper side (an upper direction) is denoted by "U" and the lower side (a lower direction) is denoted by "D" in a state in which a distal end side of the endoscope 1 in the operating device 2 is directed vertically downward. Regarding the left-right direction, the right side (a right direction) is denoted by "R" and the left side (a left direction) is denoted by "L" as viewed from an operator gripping the operating device 2. Regarding the front-rear direction, the rear side (a rear direction) is denoted by "Rr" and the front side (a front direction) is denoted by "Fr" as viewed from the operator gripping the operating device 2. The upper side in the up-down direction is also denoted by a proximal end side of the operating device 2 or of the endoscope 1, and the lower side in the up-down direction is also denoted by a distal end side of the operating device 2 or of the endoscope 1.

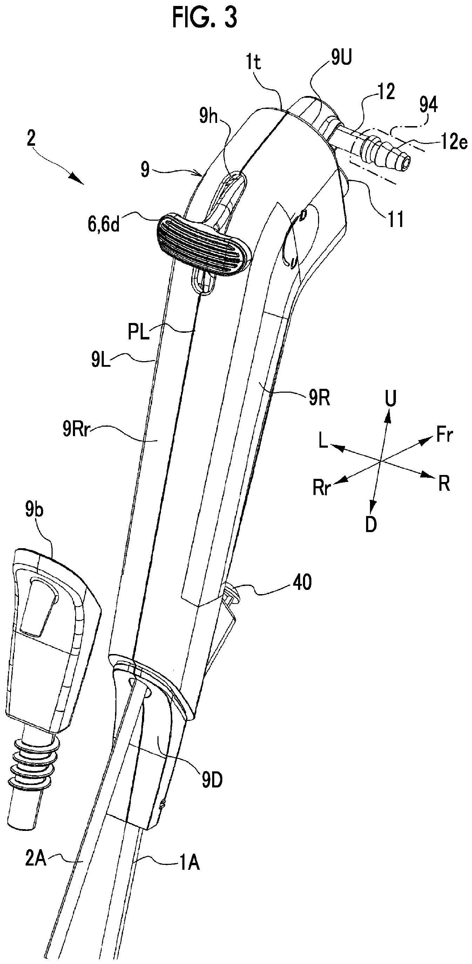

Fig. 2 is an enlarged perspective view of the operating device 2 shown in Fig. 1 as viewed obliquely from the front side. Fig. 3 is a perspective view of the operating device 2 shown in Fig. 2 as viewed obliquely from the rear side. Fig. 4 is an exploded perspective view of the operating device 2.

As shown in Figs. 2 to 4, the operating device 2 has a configuration in which each component is accommodated in a housing 9 that is elongated in the up-down direction. The housing 9 comprises a first case member 3 provided on the left side and a second case member 4 which is provided on the right side and which is stuck to the first case member 3. As will be described later, the first case member 3 and the second case member 4 are separably engaged with each other, and the first case member 3 and the second case member 4 are disengaged from each other so that the first case member 3 and the second case member 4 are separable from each other in the left-right direction at a boundary line PL. The boundary line PL is a line formed by an outer edge of a butting surface between the first case member 3 and the second case member 4 and also exists at positions where the suction button 11 and the suction nozzle 12 are provided, at a position where the introduction pipe line 40 is provided, at a position where the insertion part 1A is provided, and at a position where the cable 2A is provided, as shown in Figs. 2 to 4.

An outer surface of the housing 9 includes a right-side surface 9R provided on the right side, a left-side surface 9L provided on the left side, a rear-side surface 9Rr provided on the rear side, an upper-side surface 9U that is connected to an edge 1t provided on an upper side of the rear-side surface 9Rr and that extends obliquely toward the lower side (the distal end side of the endoscope 1), a front-side surface 9Fr connected to the upper-side surface 9U, a lower-side surface 9D that is connected to an edge provided on a lower side of the rear-side surface 9Rr, that extends obliquely toward the lower side (the distal end side of the endoscope 1), and that faces a direction opposite to the upper-side surface 9U. The rear-side surface 9Rr and the lower-side surface 9D form a first side surface. The edge 1t provided on the upper side of the rear-side surface 9Rr forms an edge provided on the proximal end side of the operating device 2. The introduction pipe line 40 is provided at a lower end part of the front-side surface 9Fr. The introduction pipe line 40 is configured to extend in a direction (obliquely front-upper direction) non-orthogonally intersecting the up-down direction and the left-right direction.

As shown in Fig. 3, the rear-side surface 9Rr is provided with a side surface opening 9h at a curved corner part provided close to the upper side thereof. A part of the bending operation portion 6 for operating the bendable portion 1b is provided so as to protrude from the side surface opening 9h. The suction nozzle 12 and the suction button 11 are provided on the upper-side surface 9U in a protruding manner so as to be arranged along the boundary line PL. The suction nozzle 12 is disposed on the proximal end side (an edge 1t side) with respect to the suction button 11.

The suction pipe 94 is connected to a nozzle distal end 12e of the suction nozzle 12. The cable 2A is pulled out from the lower-side surface 9D and is connected to the external device. The lower-side surface 9D is covered with a cover member 9b in a product state. The insertion part 1A is provided so as to protrude from a surface of the housing 9, which interconnects a lower end edge of the lower-side surface 9D, a lower end edge of the right-side surface 9R, a lower end edge of the left-side surface 9L, and a lower end edge of the front-side surface 9Fr. The cable 2A and the insertion part 1A are each disposed on the boundary line PL.

As shown in Fig. 4, the suction unit 10, a wire unit 18, the cable 2A, and the insertion part 1A are accommodated in the operating device 2. A sleeve-shaped insertion part holding portion 1g is attached to the proximal end side of the insertion part 1A by adhesion or the like.

The suction unit 10 includes the suction button 11, the suction nozzle 12, a connecting pipe 13 that connects the suction button 11 and the suction nozzle 12 to each other, a plate-shaped holding member 5 that holds the suction button 11 and the suction nozzle 12, a suction pipe line 30 that is formed of a flexible tube or the like and that extends along the up-down direction, and a branching member 50. The branching member 50 is a member that branches the insertion pipe line 20 (a pipe line through which the treatment tool is inserted) that is formed of a treatment tool channel tube or the like incorporated in the insertion part 1A of the endoscope 1 into the introduction pipe line 40 and the suction pipe line 30. In the present embodiment, the branching member 50 and the introduction pipe line 40 are integrally formed, but these may be configured to be separate bodies.

The wire unit 18 includes the bending operation portion 6, a pair of wires 6w (only a part thereof is shown in the drawing) that connect the bending operation portion 6 and the bendable portion 1b of the endoscope 1 to each other, a tubular guide member 6p through which each wire 6w is inserted, a circuit board 7, and a support member 8 that supports the guide member 6p and the circuit board 7. The support member 8 has a plate shape of which a thickness direction coincides with the left-right direction and a longitudinal direction coincides with the up-down direction.

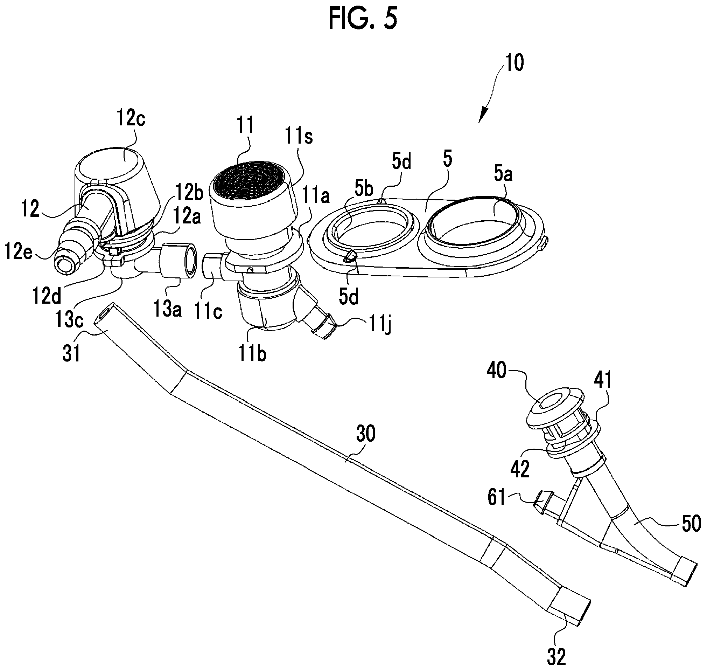

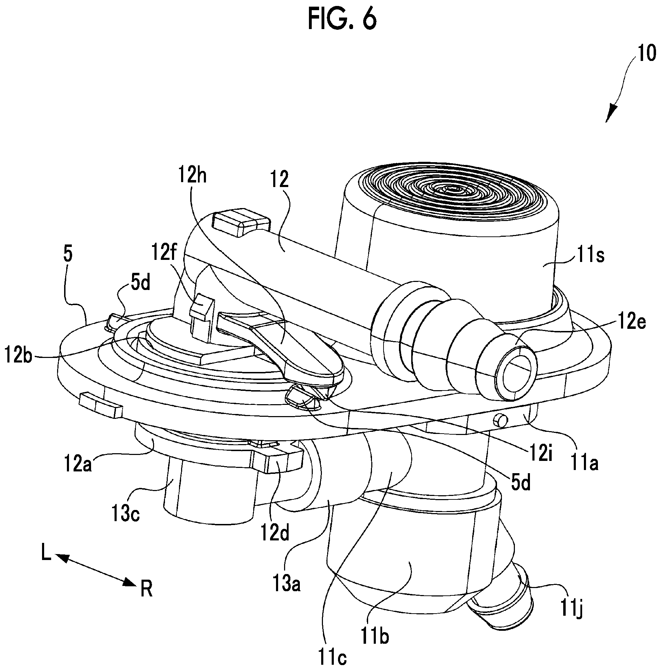

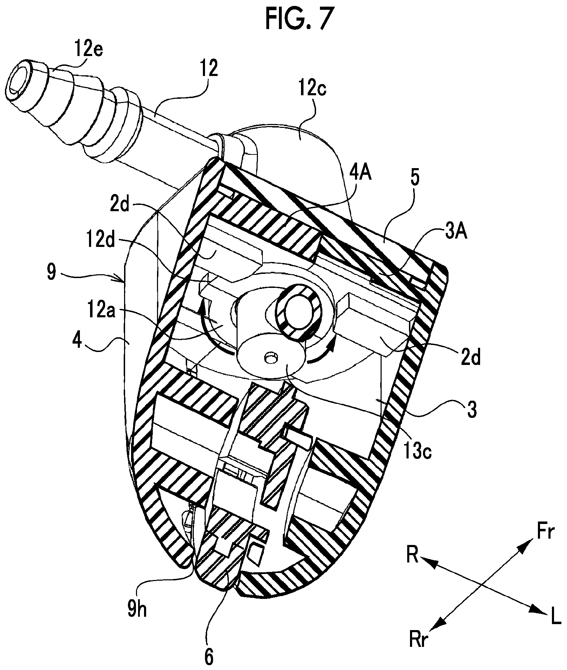

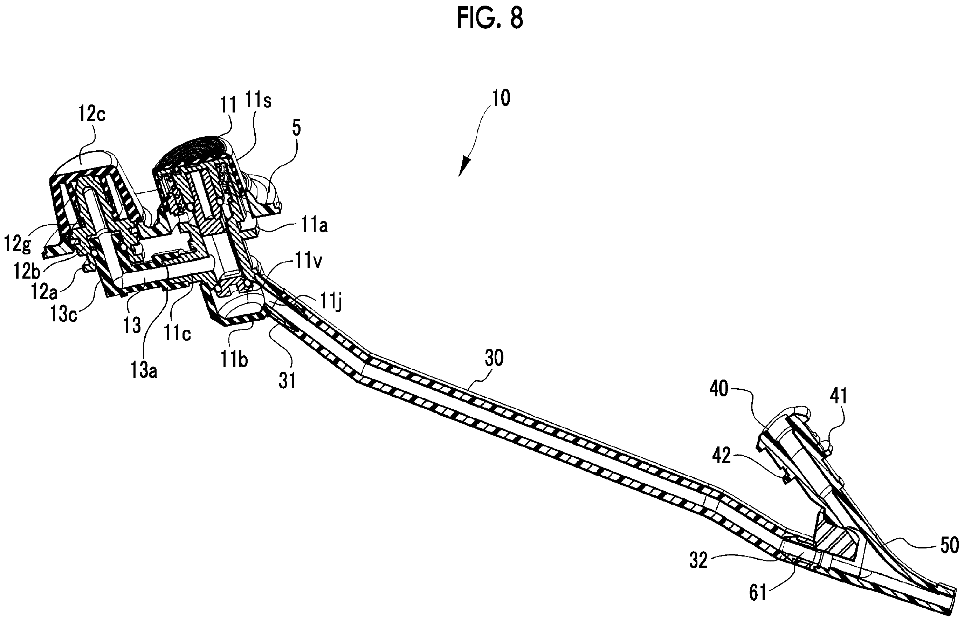

Fig. 5 is an exploded perspective view of the suction unit 10. Fig. 6 is an exploded perspective view showing a structure in which the suction nozzle 12 is incorporated into the holding member 5. Fig. 7 is a cross-sectional perspective view showing a structure that restricts a rotational movement range of the suction nozzle 12. Fig. 8 is a cross-sectional view of the suction unit 10 in an assembled state.

As shown in Fig. 8, the connecting pipe 13 has a structure having a substantially L-shape. One end 13a of the connecting pipe 13 is connected to a connection portion 11c provided on the suction button 11, and the other end 13c of the connecting pipe 13 is attached to a nozzle body 12b of the suction nozzle 12.

As shown in Fig. 5, the suction nozzle 12 comprises the nozzle body 12b that is attached to the other end 13c of the connecting pipe 13 so as to be rotationally movable, a cap member 12c that is provided so as to cover the nozzle body 12b, and a nozzle distal end 12e that protrudes to the right side from an end part of the nozzle body 12b on a side opposite to the other end 13c side of the connecting pipe 13. The nozzle body 12b is rotationally movable about the other end 13c of the connecting pipe 13 with respect to the other end 13c of the connecting pipe 13.

The nozzle body 12b is mounted in a nozzle hole 5b that is provided in the holding member 5 so as to penetrate in a thickness direction. The cap member 12c is attached after the nozzle body 12b is mounted in the nozzle hole 5b. For example, the cap member 12c is attached by engagement between a projection to be locked 12g (see Fig. 8) provided on an inner surface of the cap member 12c and a locking projection 12f (see Fig. 6) provided on the nozzle body 12b.

As shown in Fig. 6, an arm portion 12h projecting in the same direction as the nozzle distal end 12e is provided in a part of the nozzle body 12b between the holding member 5 and the nozzle distal end 12e. An engaging protrusion 12i that protrudes toward a holding member 5 side (the lower side in the drawing) is provided at a distal end of the arm portion 12h.

The holding member 5 has two holding portions 5d each of which protrudes so as to be engageable with the engaging protrusion 12i and which are provided at positions of 180 degrees around the nozzle body 12b. That is, the two holding portions 5d are disposed side by side in the left-right direction with a rotational movement shaft of the nozzle body 12b interposed therebetween. With these holding portions 5d, the direction of the nozzle distal end 12e can be held in either a state of facing the right or a state of facing the left.

Fig. 6 shows a state in which the nozzle distal end 12e is held at a position facing the right by the engagement between the engaging protrusion 12i and the right-side holding portion 5d. From the state shown in Fig. 6, in a case in which the nozzle distal end 12e is moved leftward such that the engaging protrusion 12i goes over the right-side holding portion 5d, the arm portion 12h bends so that the engaging protrusion 12i and the right-side holding portion 5d are disengaged from each other. With this, the nozzle distal end 12e can be rotated to a position facing the left. In a case in which the nozzle distal end 12e is rotated until the engaging protrusion 12i goes over the left-side holding portion 5d, the engaging protrusion 12i and the left-side holding portion 5d are engaged with each other, and the nozzle distal end 12e is held at the position facing the left.

Here, as shown in Figs. 6 and 7, a brim portion 12a having a shape in which a diameter increases in a radial direction of the nozzle body 12b is formed at a lower end of the nozzle body 12b. A restricting protrusion 12d that protrudes radially outward is provided on a distal end surface of the brim portion 12a. A protruding direction of the restricting protrusion 12d is the same as a protruding direction of the nozzle distal end 12e. As shown in Fig. 7, the restricting protrusion 12d is capable of coming into contact with protruding restricting portions 2d provided on inner surfaces of the first case member 3 and of the second case member 4, respectively. These two restricting portions 2d are disposed side by side in the left-right direction.

In the state of Fig. 6 in which the engaging protrusion 12i and the right-side holding portion 5d are engaged with each other, the restricting portion 2d provided on the second case member 4 and the restricting protrusion 12d come into contact with each other so that the nozzle distal end 12e is restricted from being further rotated toward the front side, as shown in Fig. 7. Further, in a state in which the engaging protrusion 12i and the left-side holding portion 5d are engaged with each other, the restricting portion 2d provided on the first case member 3 and the restricting protrusion 12d come into contact with each other so that the nozzle distal end 12e is restricted from being further rotated toward the front side. As described above, the suction nozzle 12 is rotationally movable between a state of facing one side in the thickness direction of the hand gripping the operating device 2 (for example, a state of facing an inner side of a left hand as shown in Fig. 2) and a state of facing the other side in the thickness direction (a state of facing the back of the hand as opposed to the state of Fig. 2).

As shown in Fig. 8, the suction button 11 comprises a button body 11b including an operation element 11s which has an internal cavity and which can be pushed, and an opening and closing valve 11v provided inside the button body 11b. The button body 11b is mounted in a button hole 5a (see Fig. 5) provided in the holding member 5 so as to penetrate in the thickness direction. The button body 11b has the tubular connection portion 11c and a connection portion 11j that are connected to the inside of the button body 11b and that are provided so as to protrude. An upper end part 31 of the suction pipe line 30 is connected to the connection portion 11j. A lower end part 32 of the suction pipe line 30 is connected to a connection portion 61 of a connecting member 60 provided in the branching member 50. A brim portion 11a is formed on an inner side of the button body 11b with respect to the holding member 5. The connection portion 11c and the connection portion 11j are provided on the inner side with respect to the brim portion 11a.

The operation element 11s of the suction button 11 is pushed so that the opening and closing valve 11v is opened, and in this state, the nozzle distal end 12e of the suction nozzle 12 and the suction pipe line 30 communicate with each other via the suction button 11 and the connecting pipe 13. In a state in which the operation element 11s of the suction button 11 is not pushed, the opening and closing valve 11v is closed, and the nozzle distal end 12e of the suction nozzle 12 and the suction pipe line 30 do not communicate with each other.

In the suction unit 10 configured as described above, respective suction system components of the holding member 5, the suction button 11, the suction nozzle 12, the connecting pipe 13, the suction pipe line 30, and the branching member 50 are completed by being assembled only by fitting, press-fitting, or engaging without using adhesion, screwing, or the like.

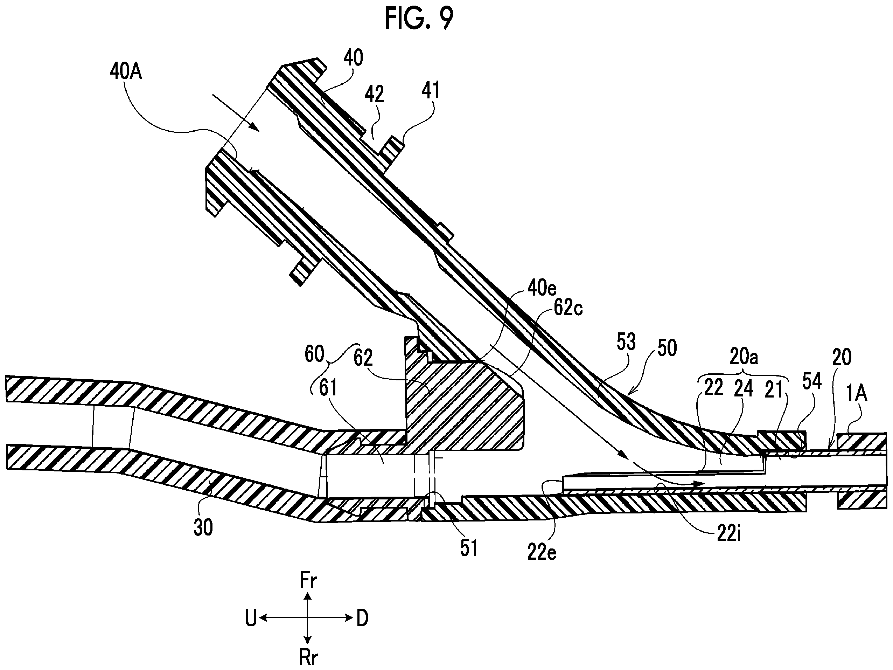

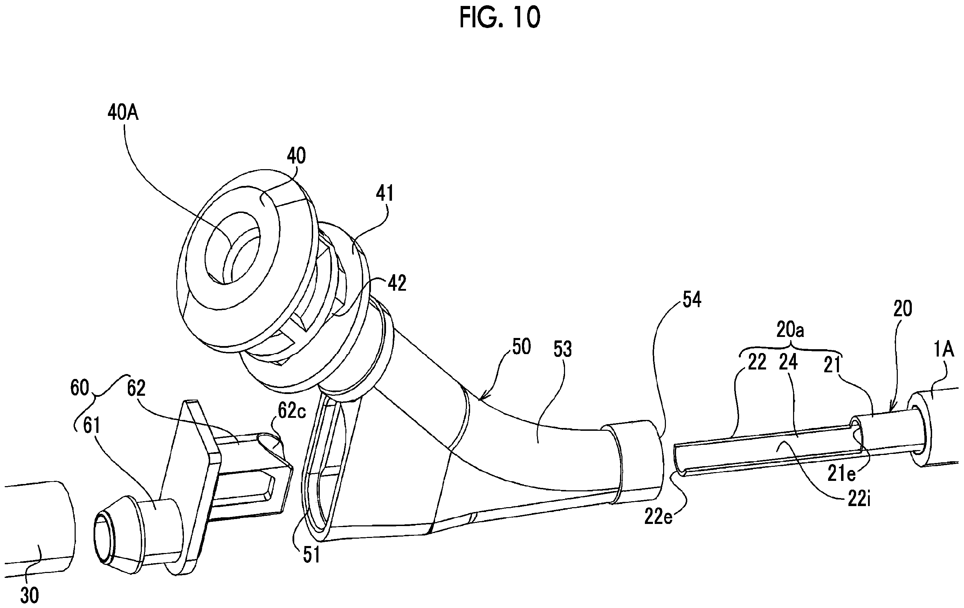

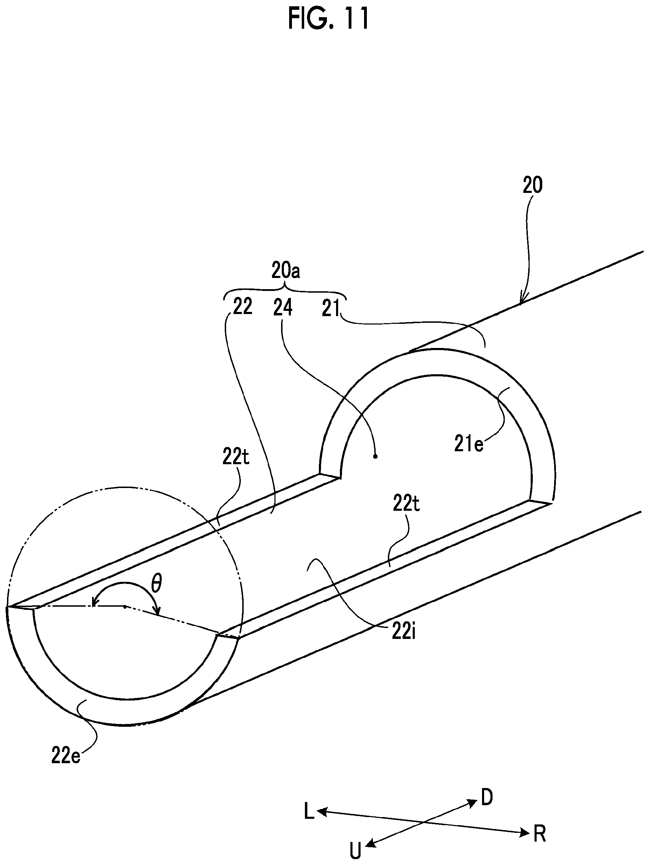

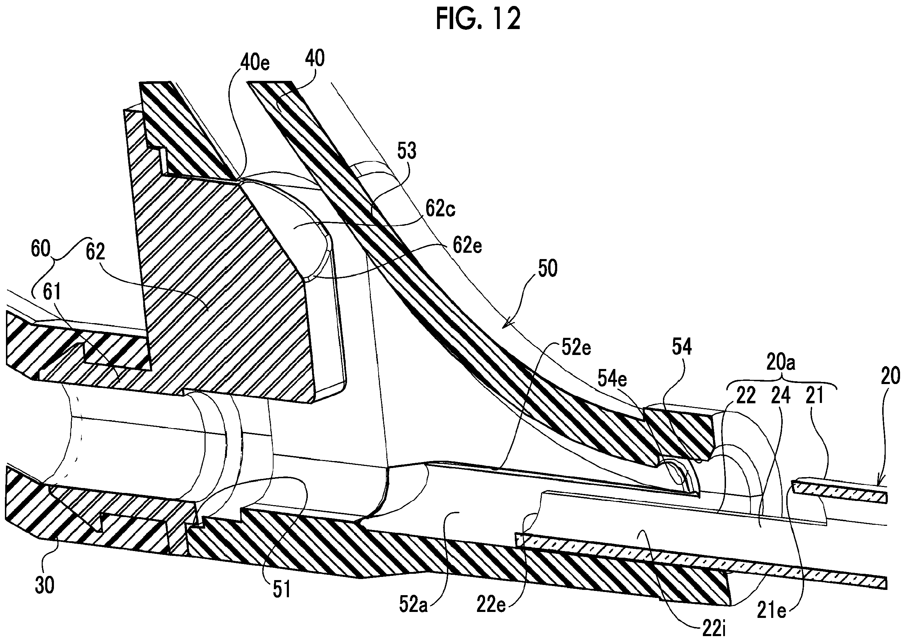

Fig. 9 is an enlarged cross-sectional view of the periphery of the branching member 50. Fig. 9 shows a cut surface perpendicular to the left-right direction. Fig. 10 is an exploded perspective view of each component shown in Fig. 9. Fig. 11 is a perspective view showing a proximal end portion 20a of the insertion pipe line 20. Fig. 12 is a partially enlarged cross-sectional perspective view of the branching member 50.

The branching member 50 comprises a body part 53 having an internal space that communicates in a Y shape, and the connecting member 60 that is attachable to and detachable from the body part 53. The body part 53 has a treatment tool introduction opening 40A through which the treatment tool 90 can be inserted, an insertion pipe line side opening 54 into which the insertion pipe line 20 is inserted, and a fitting opening 51 to which the connecting member 60 is fitted, and has the Y-shaped internal space that interconnects these three openings. Figs. 9 and 12 show a branch position 40e of the internal space in the body part 53. The introduction pipe line 40 is formed by a tubular part extending from the branch position 40e to the treatment tool introduction opening 40A. A tubular part extending from the branch position 40e to the fitting opening 51 forms a connecting pipe line capable of connecting the insertion pipe line 20 and the suction pipe line 30 to each other. A flange portion 41 is provided on an outer peripheral surface of the body part 53 forming the introduction pipe line 40 at a position close to the treatment tool introduction opening 40A, and a groove portion 42 is provided between the flange portion 41 and the treatment tool introduction opening 40A. An axis of the introduction pipe line 40 extends in a direction intersecting the up-down direction and the front-rear direction, and extends in the front-upper direction in the example of Fig. 9.

The connecting member 60 connects the internal space of the body part 53 and the suction pipe line 30, and comprises the tubular connection portion 61 to which the suction pipe line 30 is connected and a guide portion 62 that is formed integrally with the connection portion 61 and that guides the movement of the treatment tool 90 inserted through the introduction pipe line 40. An axis of the connection portion 61 coincides with the up-down direction. The guide portion 62 is fitted to the fitting opening 51 so that the connecting member 60 is mounted on the body part 53. As shown in Figs. 9 and 12, in a state in which the guide portion 62 is fitted to the fitting opening 51, a guide surface 62c of the guide portion 62 is configured not to protrude from an inner wall surface of the introduction pipe line 40 when viewed in an axial direction of the introduction pipe line 40. Specifically, the guide surface 62c is an arc-shaped curved surface and has a shape inclined along the axis of the introduction pipe line 40. Further, as shown in Fig. 12, an edge 62e of the guide surface 62c has a rounded configuration (a chamfered configuration).

The insertion pipe line 20 is configured to extend to the distal end of the insertion part 1A, and has an axial direction that coincides with the up-down direction. The proximal end side of the insertion pipe line 20 is provided outside the insertion part 1A. As shown in Fig. 9, the insertion pipe line 20 provided outside the insertion part 1A is inserted into the branching member 50 from the insertion pipe line side opening 54. The proximal end portion 20a of the insertion pipe line 20 inserted into the branching member 50 includes a tubular first part 21 and a second part 22 provided on the proximal end side with respect to the first part 21. It is preferable that a length of the second part 22 in the up-down direction is larger than a length of the first part 21 in the up-down direction. An opening 24 facing an introduction pipe line 40 side (in other words, the front side) is formed in an outer peripheral surface of the second part 22.

It is preferable that an inner peripheral surface 22i of the second part 22, which is exposed from the opening 24, intersects an extension line of the axis of the introduction pipe line 40. In addition, it is preferable that a position of an end edge 22e provided on a proximal end side of the second part 22 in the axial direction (synonymous with the up-down direction) of the insertion pipe line 20 is located on a suction pipe line 30 side with respect to an intersection position between the extension line of the axis of the introduction pipe line 40 and the inner peripheral surface 22i. With such a configuration, in a case in which the treatment tool 90 is inserted from the introduction pipe line 40 (an insertion trajectory is indicated by arrows in Fig. 9), a distal end of the treatment tool 90 comes into contact with the inner peripheral surface 22i and is guided so as to face an inside of the first part 21. The inner peripheral surface 22i extends to the upper side with respect to the intersection position with the extension line of the axis of the introduction pipe line 40 so that the distal end of the treatment tool 90 can be prevented from coming into contact with a boundary part between the end edge 22e of the proximal end portion 20a of the insertion pipe line 20 and an inner peripheral wall of the body part 53. The treatment tool 90 does not come into contact with the boundary part, so that the treatment tool 90 can be smoothly taken in and out.

In addition, in a case in which the treatment tool 90 is taken in and out, the movement of the treatment tool 90 can be guided by the guide surface 62c of the connecting member 60. Further, in a case in which the treatment tool 90 is pulled out, the guide portion 62 can prevent the treatment tool 90 from coming into contact with the branch position 40e, which is the end of the introduction pipe line 40. As described above, with the configuration of the branching member 50, the treatment tool 90 can be smoothly taken in and out.

The branching member 50 is configured such that the body part 53 and the connecting member 60 are separate bodies, but these may be integrally molded. The body part 53 and the connecting member 60 are separate bodies, so that it is possible to increase the degree of freedom in designing the guide surface 62c. In addition, the branching member 50 can be easily manufactured.

As shown in Fig. 11, the proximal end portion 20a of the insertion pipe line 20 is formed by partially cutting a proximal end of the cylindrical insertion pipe line 20, which is conventionally known. In Fig. 11, a cut surface of the first part 21, which is exposed by this cutting and is provided on a second part 22 side, is shown as an edge surface 21e. In addition, a cut surface of the second part 22 exposed by this cutting is shown as an edge surface 22t. The edge surface 21e is a surface perpendicular to the axial direction of the insertion pipe line 20. The edge surface 22t is a surface parallel to the up-down direction and to the left-right direction.

As shown in Fig. 12, the insertion pipe line side opening 54 of the branching member 50 is configured to have a diameter slightly larger than that of a deep side (the upper side) thereof, and a deepest portion into which the first part 21 is inserted has a contact surface 54e formed by a difference in inner diameter with the deep side. The contact surface 54e is a surface perpendicular to the axial direction of the insertion pipe line 20. The contact surface 54e and the edge surface 21e of the proximal end portion 20a come into contact with each other so that an insertion depth of the insertion pipe line 20 with respect to the branching member 50 is defined.

In addition, a large-diameter stepped portion 52a having a part of an inner peripheral surface formed in a slightly larger diameter is provided inside the body part 53 on the deep side with respect to the contact surface 54e in order to receive the second part 22. The large-diameter stepped portion 52a forms, in the body part 53, a stepped surface 52e that comes into contact with the edge surface 22t of the insertion pipe line 20 in a case in which the second part 22 is received in the large-diameter stepped portion 52a. The stepped surface 52e is a surface parallel to the up-down direction and to the left-right direction. The stepped surface 52e and the edge surface 22t come into contact with each other so that the proximal end portion 20a of the insertion pipe line 20 is restricted from radially rotating.

It is preferable that, in a case in which an opening angle θ (see Fig. 11) from the center of the insertion pipe line 20 is defined as viewed in the axial direction of the insertion pipe line 20, the size of the opening 24 of the insertion pipe line 20 is, for example, within a range of "270 degrees ≥ θ ≥ 180 degrees".

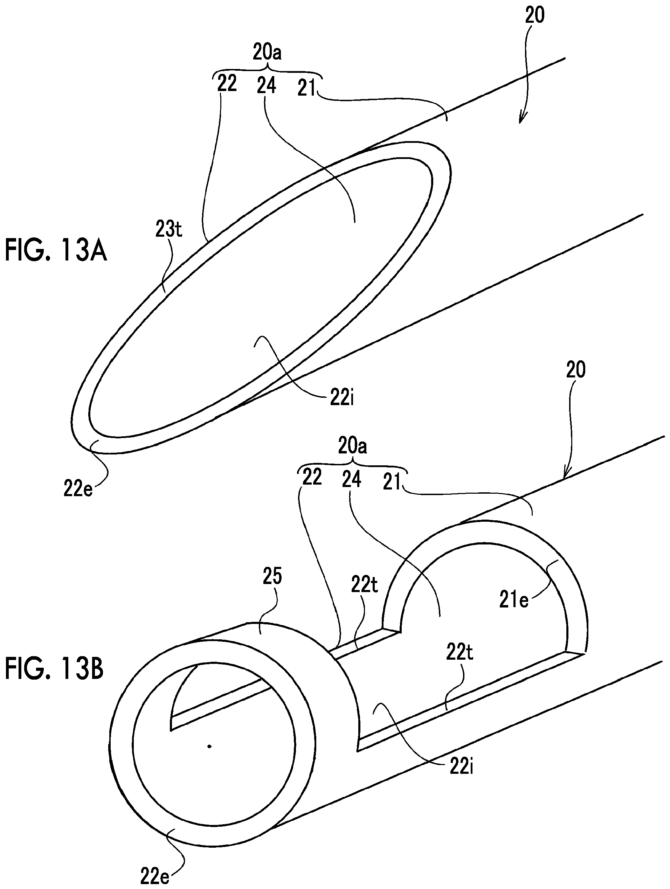

The shape of the opening 24 is not limited to the shape shown in Fig. 11, and various shapes can be employed. Figs. 13A and 13B are perspective views showing two modification examples of the proximal end portion 20a of the insertion pipe line 20. For example, as shown in Fig. 13A, a configuration may be employed in which a distal end part is obliquely cut at the proximal end portion 20a of the insertion pipe line 20. That is, a cut surface 23t of the second part 22 may be inclined in an elliptical shape. Alternatively, as shown in Fig. 13B, a configuration may be employed in which the opening 24 formed by leaving an annular distal end portion 25 having an annular shape at the distal end part is provided in the proximal end portion 20a of the insertion pipe line 20.

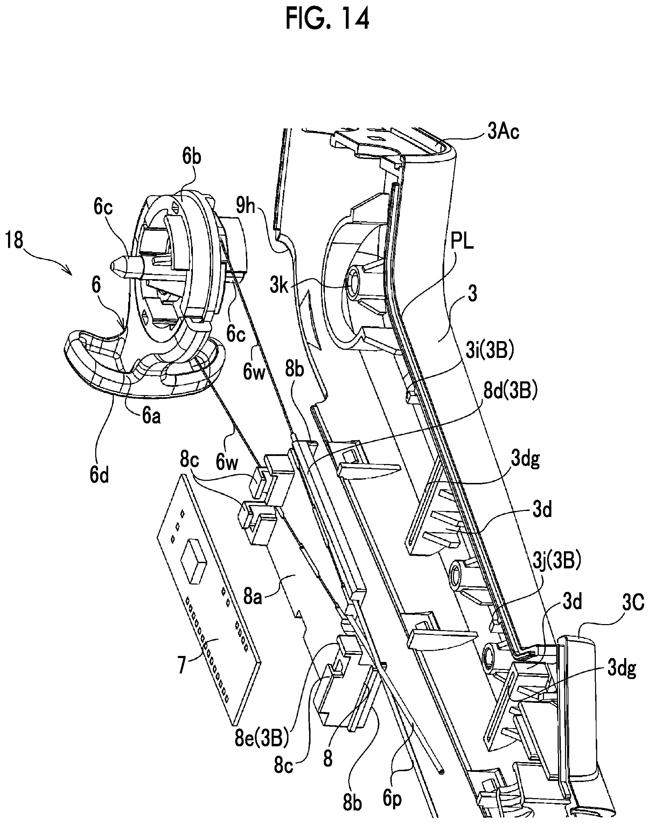

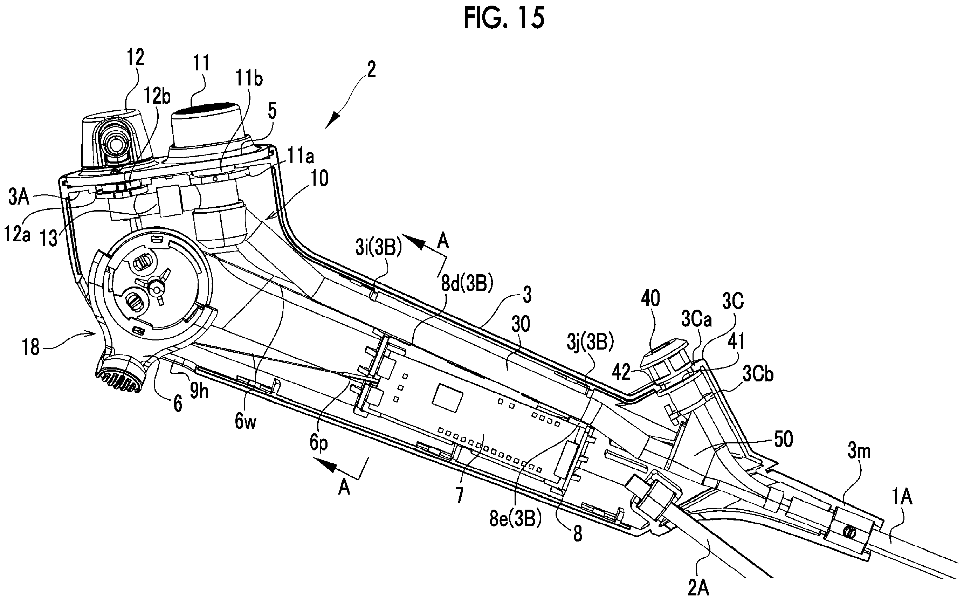

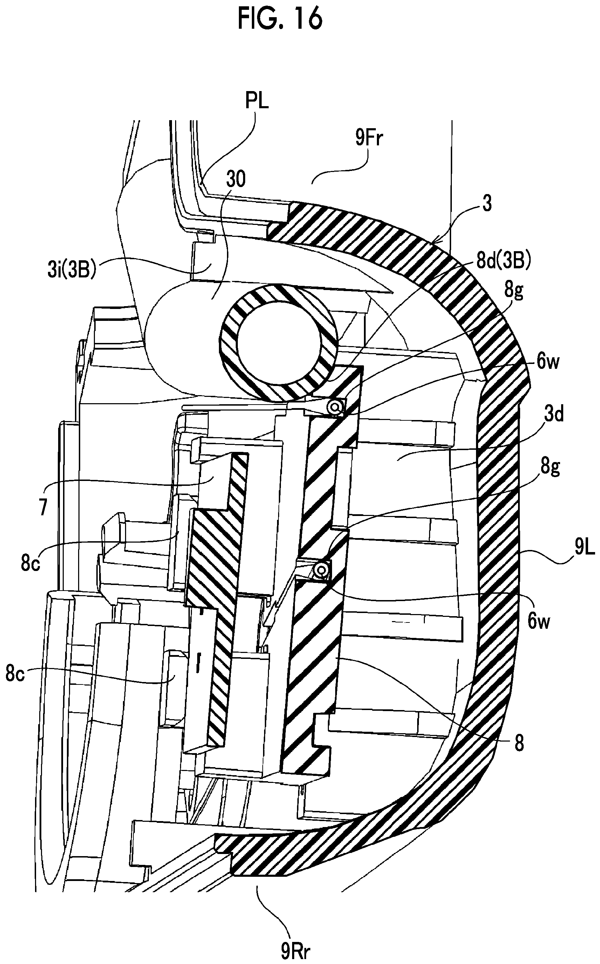

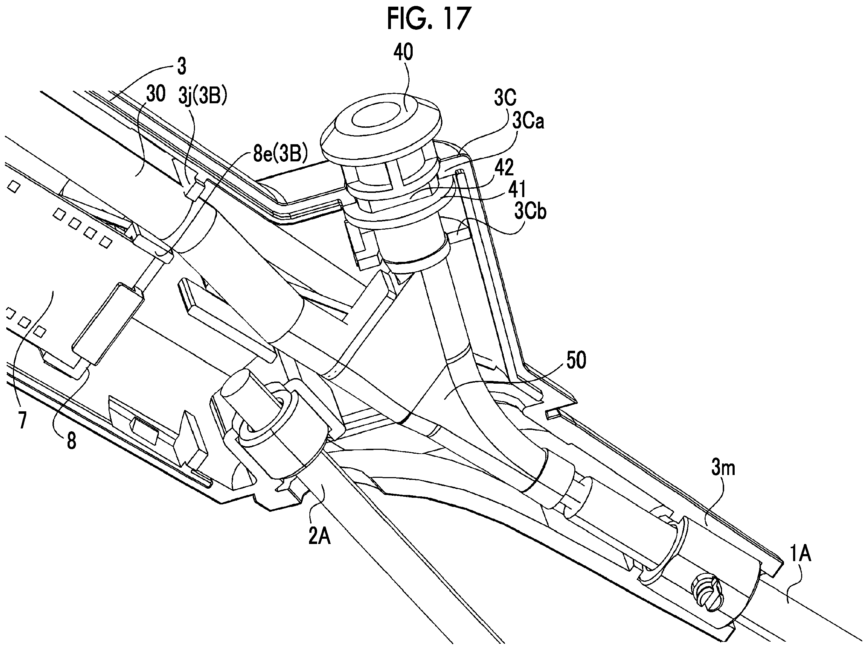

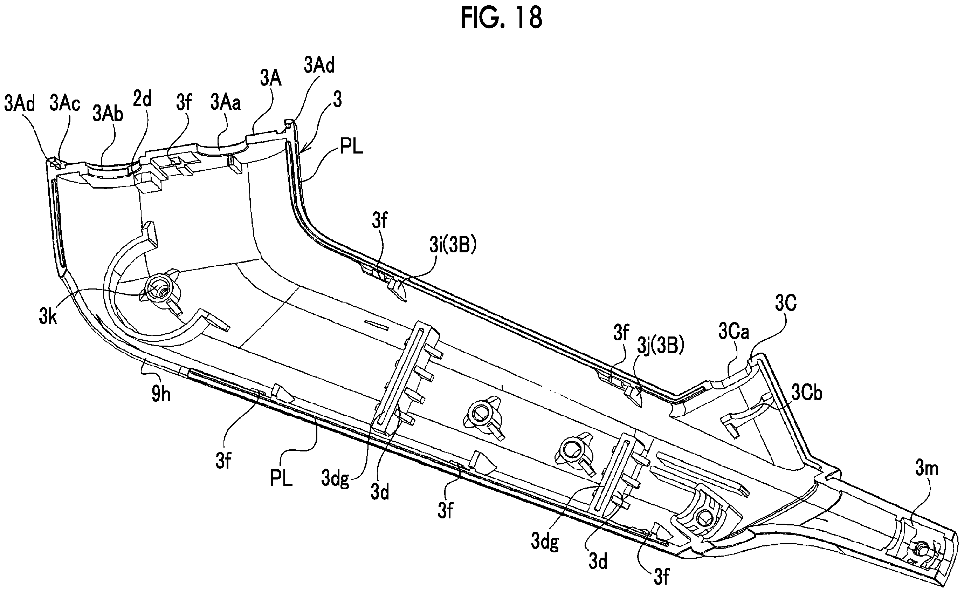

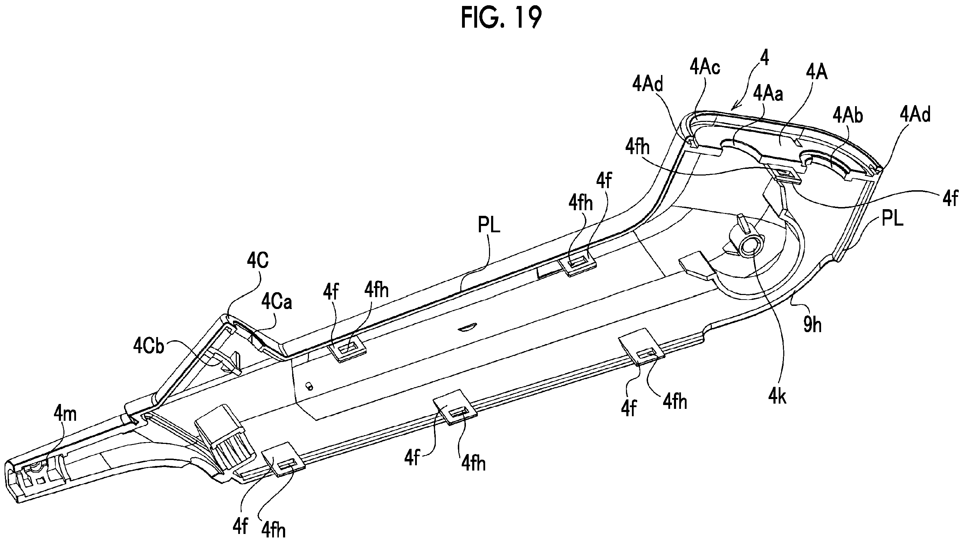

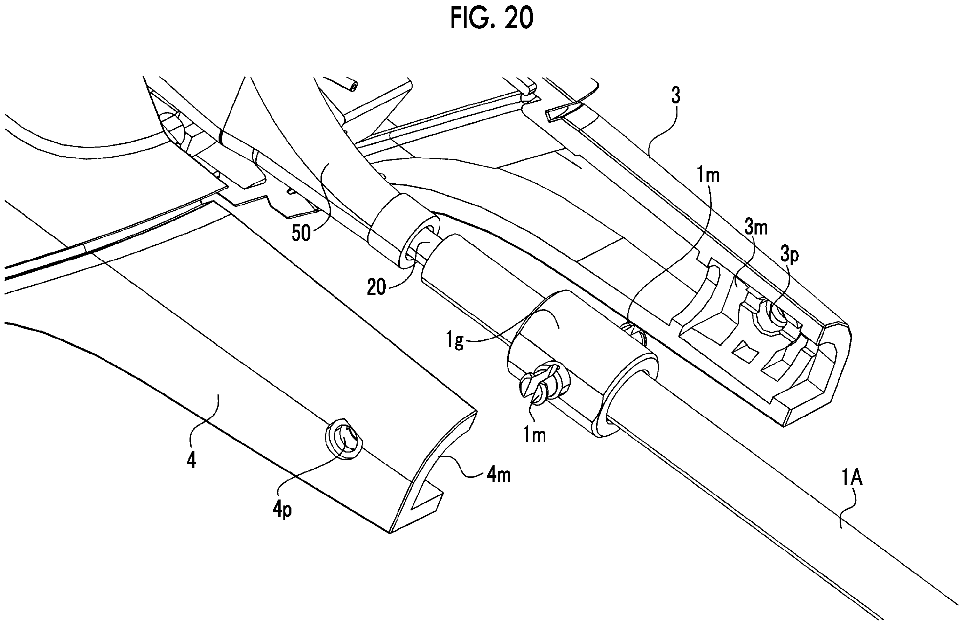

Fig. 14 is an enlarged perspective view showing an attachment structure of the bending operation portion 6 and of the circuit board 7. Fig. 15 is an exploded perspective view showing a state in which each component is incorporated into the first case member 3. Fig. 16 is a cross-sectional view taken along line A-A of Fig. 15. Fig. 17 is an enlarged perspective view of a lower part in Fig. 15. Fig. 18 is a perspective view showing the inner surface of the first case member 3. Fig. 19 is a perspective view showing the inner surface of the second case member 4. Fig. 20 is an enlarged exploded perspective view of a lower end part of the housing 9.

As shown in Fig. 14, the bending operation portion 6 comprises a pulley 6b that is rotatable about a rotation support shaft 6c, and an operation lever portion 6a extending from a part of an outer peripheral surface of the pulley 6b (specifically, a half part of the outer peripheral surface) in a rotation radius direction of the pulley 6b. A finger rest portion 6d is provided at a distal end of the operation lever portion 6a. The pair of wires 6w are attached to the pulley 6b at an interval. The bending operation portion 6 is disposed such that only the operation lever portion 6a protrudes from the side surface opening 9h to the outside.

The wire 6w is movably supported via the guide member 6p supported in a guide groove 8g (see Fig. 16) provided in a plane portion 8a of the support member 8. Further, the circuit board 7 is locked to support hooks 8c which are provided at both ends in the longitudinal direction of the support member 8 in a protruding manner. The circuit board 7 may further adhere to the support member 8 with an adhesive.

Each component is incorporated into the housing 9 by, first, covering the first case member 3 with the second case member 4 in a state in which each component is mounted on the first case member 3 and by engaging these with each other. Specifically, first, the wire unit 18, the cable 2A, the suction unit 10, and the insertion part 1A are connected to form one unit. The connection between the branching member 50 and the insertion pipe line 20 is performed, for example, by applying an adhesive onto an outer peripheral surface of the first part 21 of the proximal end portion 20a, by inserting the proximal end portion 20a in this state into the branching member 50, and by adhering the first part 21 to an inner peripheral wall of the branching member 50.

Next, as shown in Fig. 15, the above-described units are mounted on the first case member 3. Specifically, as shown in Fig. 14, the rotation support shaft 6c of the bending operation portion 6 is incorporated so as to be supported by a rotation support portion 3k provided on the inner surface of the first case member 3. Further, the support member 8 is incorporated such that fitting end portions 8b provided at both end parts in the longitudinal direction thereof are fitted into fixing grooves 3dg of a pair of attachment protrusions 3d provided on the inner surface of the first case member 3. The support member 8 may further adhere to the first case member 3 with an adhesive.

The suction unit 10 is mounted on a first mounting portion 3A (see Fig. 18), which corresponds to the suction button 11, the suction nozzle 12, and the holding member 5 and is provided in the first case member 3, on a second mounting portion 3B (see Fig. 18), which corresponds to the suction pipe line 30 and is provided in at least the inner surface of the first case member 3, and on a third mounting portion 3C (see Fig. 18), which corresponds to the branching member 50 and is provided in the first case member 3. Further, the proximal end part of the insertion part 1A is mounted in the first case member 3 such that a part of the insertion part holding portion 1g is fitted into the first case member 3.

As shown in Fig. 20, a holding end portion 3m is provided on an inner surface of a lower end of the first case member 3. A holding end portion 4m is provided on an inner surface of a lower end of the second case member 4. The insertion part holding portion 1g is provided with locking projections 1m that have each a slit and that protrude in the left-right direction. Meanwhile, the holding end portion 3m is provided with a fitting hole 3p into which the locking projection 1m is fitted. The holding end portion 4m is provided with a fitting hole 4p into which the locking projection 1m is fitted. Therefore, the proximal end part of the insertion part 1A is mounted in the first case member 3 by the fitting between the left-side locking projection 1m and the fitting hole 3p.

The first mounting portion 3A is a wall surface forming the upper-side surface 9U. As shown in Fig. 18, the wall surface has a semi-circular button notch 3Aa that receives the button body 11b and a nozzle notch 3Ab that receives the nozzle body 12b which are provided along the boundary line PL. In addition, a wall portion 3Ac that rises outward from a peripheral edge excluding the boundary line PL is provided on the wall surface. An engaging groove 3Ad that is engaged with a protrusion provided on a side surface of the holding member 5 is provided in an inner surface of the wall portion 3Ac. The suction unit 10 is mounted on the first mounting portion 3A such that the holding member 5 is disposed in a part surrounded by the wall portion 3Ac and the brim portion 11a and the brim portion 12a enter the inner side of the first mounting portion 3A. At this time, the protrusion provided on the side surface of the holding member 5 is engaged with the engaging groove 3Ad of the wall portion 3Ac so that the movement of the holding member 5 in the thickness direction is restricted.

As shown in Figs. 15 to 17, the second mounting portion 3B is composed of a total of four members, that is, two pipe line protrusions 3i and 3j formed on an inner side of the front-side surface 9Fr of the first case member 3, and a side piece portion 8d and a side piece portion 8e provided on a side on the front side of the support member 8 mounted on the first case member 3. That is, the second mounting portion 3B comes into contact with an outer peripheral surface of the suction pipe line 30 at a plurality of places to hold the suction pipe line 30. Here, a part of the support member 8 is used to form the second mounting portion 3B, but this is not essential.

The third mounting portion 3C is a wall surface that forms a part of the front-side surface 9Fr of the housing 9 on the lower side. As shown in Fig. 18, the wall surface is provided with a semi-circular pipe line notch 3Ca that receives the introduction pipe line 40 of the branching member 50. Further, a rib 3Cb having a semi-circular notch that receives a termination portion of the introduction pipe line 40 is provided on the inner surface of the first case member 3 at a position on the inner side with respect to the pipe line notch 3Ca. As shown in Fig. 17, the flange portion 41 of the branching member 50 is mounted so as to be fitted to the inner side with respect to the pipe line notch 3Ca so that the groove portion 42 of the branching member 50 is received in the pipe line notch 3Ca, whereby the branching member 50 is mounted on the first case member 3.

As described above, the above-described units are incorporated into the first case member 3 and temporarily fixed. After that, the first case member 3 is combined with the second case member 4, whereby each of the suction button 11, the suction nozzle 12, and the introduction pipe line 40, out of the above-described units, is partially sandwiched between the first case member 3 and the second case member 4 and the positions in the housing 9 are fixed.

The second case member 4 comprises a wall surface structure capable of partially sandwiching the suction unit 10 by facing each of the first mounting portion 3A and the third mounting portion 3C. As shown in Fig. 19, a button notch 4Aa that receives the button body 11b, a nozzle notch 4Ab that receives the nozzle body 12b, and a wall portion 4Ac including an engaging groove 4Ad are provided in the second case member 4 at a position corresponding to the first mounting portion 3A. Therefore, in a case in which the second case member 4 is engaged with the first case member 3, the button body 11b of the suction button 11 is sandwiched between the button notch 3Aa and the button notch 4Aa, and the nozzle body 12b of the suction nozzle 12 is sandwiched between the nozzle notch 3Ab and the nozzle notch 4Ab, and the holding member 5 is sandwiched between the wall portion 3Ac and the wall portion 4Ac. Further, the holding member 5 is engaged with the engaging groove 3Ad and the engaging groove 4Ad so that the movement of the holding member 5 in the thickness direction is restricted.

In addition, a third mounting portion facing portion 4C that is provided with a semi-circular pipe line notch 4Ca that receives the introduction pipe line 40 of the branching member 50 and a rib 4Cb having a semi-circular notch that receives the termination portion of the introduction pipe line 40 is provided in the second case member 4 at a position corresponding to the third mounting portion 3C. Therefore, in a case in which the second case member 4 is engaged with the first case member 3, the introduction pipe line 40 is sandwiched between the pipe line notch 3Ca and the pipe line notch 4Ca.

In addition, a rotation support portion 4k capable of being engaged with the rotation support shaft 6c of the bending operation portion 6 is provided in the second case member 4 at a position corresponding to the rotation support portion 3k. Therefore, in a case in which the second case member 4 is engaged with the first case member 3, the bending operation portion 6 is sandwiched between the rotation support portion 3k and the rotation support portion 4k.

As shown in Fig. 18, six engaging portions 3f protruding toward a second case member 4 side are provided on the inner surface of the first case member 3 along the boundary line PL. As shown in Fig. 19, six portions to be engaged 4f protruding toward a first case member 3 side are provided on the inner surface of the second case member 4 along the boundary line PL. The portion to be engaged 4f is configured to be appropriately bent by a plate-shaped protruding piece having an engaging hole 4fh provided on a distal end side thereof. Therefore, the boundary line PL between the first case member 3 and the second case member 4 is butted so that the engaging portions 3f are fitted into the engaging holes 4fh and the first case member 3 and the second case member 4 are engaged and stuck to each other.

The insertion part 1A is not only sandwiched by an engaging force between the first case member 3 and the second case member 4. As shown in Fig. 20, the position of the insertion part 1A in the housing 9 is more firmly fixed by the fitting between the insertion part holding portion 1g and the first case member 3 and by the fitting between the insertion part holding portion 1g and the second case member 4. A load is likely to be applied to the vicinity of the insertion part holding portion 1g because of the routing of the insertion part 1A. Therefore, the insertion part holding portion 1g is fitted to the case members, so that it is possible to prevent the stress on the insertion part 1A from being transmitted upstream of the insertion part holding portion 1g. Further, as shown in Fig. 20, the holding end portion 3m is provided with ribs having contact surfaces that come into contact with an upper end surface and a lower end surface of the insertion part holding portion 1g. Similarly, as shown in Fig. 19, the holding end portion 4m is provided with ribs having contact surfaces that come into contact with the upper end surface and the lower end surface of the insertion part holding portion 1g. These four ribs and the both-end surfaces of the insertion part holding portion 1g in the up-down direction come into contact with each other, so that a load in the axial direction of the insertion part 1A can be received by the housing 9. As a result, it is possible to prevent the stress from being transmitted to members provided upstream of the insertion part holding portion 1g.

It is preferable that each component of the operating device 2, which has been described above, excluding the cable 2A is made of a resin except for the wires 6w and the guide members 6p. With this, the manufacturing cost and the weight of the operating device 2 can be reduced. Since cleaning is not required for the disposable endoscope, these components are each made of a resin so that the manufacturing cost can be reduced.

As described above, with the endoscope 1, the suction button 11, the suction nozzle 12, and the connecting pipe 13, which tend to have a complicated structure, are unitized, and the suction button 11 and the suction nozzle 12 are partially sandwiched between the first case member 3 and the second case member 4 so that the positions of the suction button 11 and of the suction nozzle 12 in the housing 9 are fixed. Therefore, the operating device 2 can be easily assembled, and the manufacturing cost can be reduced. Further, the branching member 50 is partially sandwiched between the first case member 3 and the second case member 4 so that the position of the branching member 50 in the housing 9 is fixed. As a result, the operating device 2 can be more easily assembled, and the manufacturing cost can be further reduced.

Further, with the endoscope 1, the support member 8 is supported only by the first case member 3 out of the first case member 3 and the second case member 4. That is, the second case member 4 is not provided with a structure for supporting the support member 8. Therefore, the structure of the second case member 4 can be simplified, and the manufacturing cost can be reduced.

Further, with the endoscope 1, the suction nozzle 12 and the suction button 11 are disposed on the same side surface and are arranged linearly along the boundary line PL. Therefore, operability related to the suction operation can be improved. In particular, since the suction nozzle 12 is disposed on the proximal end side of the endoscope 1 with respect to the suction button 11, the suction pipe 94 mounted on the suction nozzle 12 can be handled at a position away from a region in which an operation component, such as the suction button 11, is disposed, and good operability can be obtained. In addition, since the suction nozzle 12 and the suction button 11 are disposed on a substantially back surface of the bending operation portion 6, interference between the suction operation and the bending operation can be prevented, and the operability can be improved. Further, a surface from which the cable 2A is pulled out and a surface in which the suction nozzle 12 is provided are located at positions separated from each other and face directions opposite to each other. With this, interference between the cable 2A and the suction pipe 94 can be prevented, and the operability can be improved. Further, since the suction nozzle 12 is rotationally movable, regardless of whether the hand gripping the operating device 2 is the right hand or the left hand, the suction nozzle 12 can be directed in a direction suitable for a direction of the hand, and the suction pipe 94 connected to the suction nozzle 12 can be handled in a direction that is easy to handle. As a result, the operability related to the suction operation can be improved.

In the endoscope 1, the holding member 5 in the suction unit 10 is not essential and may be omitted. Further, the suction unit 10 may have a configuration in which the holding member 5 is sandwiched between the first case member 3 and the second case member 4 and one or both of the suction button 11 and the suction nozzle 12 are not sandwiched between the first case member 3 and the second case member 4.

As described above, at least the following matters are described in the present specification. It should be noted that the constituent elements and the like corresponding to the above-described embodiment are shown in parentheses, but the present invention is not limited thereto.
(1) An endoscope comprising:
   an insertion pipe line (the insertion pipe line 20) through which a treatment tool is inserted;
   a suction pipe line (the suction pipe line 30);
   an introduction pipe line (the introduction pipe line 40) into which the treatment tool is introduced; and
   a branching member (the branching member 50) that branches the insertion pipe line into the introduction pipe line and the suction pipe line,
   in which a proximal end portion (the proximal end portion 20a) of the insertion pipe line is inserted into the branching member,
   the proximal end portion includes a first part (the first part 21) and a second part (the second part 22) that is provided at a proximal end side with respect to the first part, and
   an opening (the opening 24) facing an introduction pipe line side is formed in an outer peripheral surface of the second part.
   According to (1), in a case in which the treatment tool is inserted into the introduction pipe line, the treatment tool is brought into contact with the inner peripheral surface of the second part exposed from the opening and the treatment tool is moved along the inner peripheral surface, whereby the treatment tool can be inserted into the insertion pipe line. In this way, the treatment tool can be moved into the insertion pipe line without straddling the boundary between the branching member and the insertion pipe line, and the treatment tool can be smoothly moved. Similarly, in a case in which the treatment tool is pulled out, the treatment tool can also be smoothly moved.
(2) The endoscope according to (1),
   in which an inner peripheral surface (the inner peripheral surface 22i) of the second part intersects an extension line of an axis of the introduction pipe line.
   According to (2), the treatment tool can be moved to the insertion pipe line without straddling the boundary between the branching member and the insertion pipe line, and the treatment tool can be smoothly moved.
(3) The endoscope according to (2),
   in which a position of an end edge (the end edge 22e) of the second part in an axial direction of the insertion pipe line is located at a suction pipe line side with respect to an intersection position between the extension line of the introduction pipe line and the inner peripheral surface.
   According to (3), the treatment tool can be moved to the insertion pipe line without straddling the boundary between the branching member and the insertion pipe line, and the treatment tool can be smoothly moved.
(4) The endoscope according to any one of (1) to (3),
   in which the branching member includes a contact surface (the contact surface 54e) that comes into contact with an edge surface (the edge surface 21e) at a second part side of the first part.
   According to (4), positioning of the insertion pipe line with respect to the branching member can be easily performed.
(5) The endoscope according to (4),
   in which the contact surface is a surface perpendicular to an axial direction of the insertion pipe line.
   According to (5), the edge surface of the first part comes into surface contact with the contact surface and is reliably positioned. In addition, since the structure can be easily realized, the manufacturing cost can be reduced.
(6) The endoscope according to any one of (1) to (5),
   in which the branching member includes a body part (the body part 53) that includes the introduction pipe line and an opening (the insertion pipe line side opening 54) through which the insertion pipe line is inserted, and a connecting member (the connecting member 60) that connects the body part and the suction pipe line to each other.
   According to (6), the body part can be easily manufactured and the manufacturing cost can be reduced as compared with a configuration in which the connecting member and the body part are integrated.
(7) The endoscope according to (6),
   in which the connecting member includes a connection portion (the connection portion 61) to which the suction pipe line is connected and a guide portion (the guide portion 62) that guides a movement of the treatment tool inserted into the introduction pipe line.
   According to (7), since the guide function of the treatment tool can be fulfilled by the shape of the connecting member different from the body part, the degree of freedom in the shape of the guide portion is increased and the guide portion is easily formed. As a result, the structure of the body part can be simplified.
(8) The endoscope according to (7),
   in which the guide portion has a guide surface (the guide surface 62c) along the introduction pipe line, and an edge (the edge 62e) of the guide surface is rounded.
   According to (8), since the treatment tool is guided into the introduction pipe line by the rounded part of the guide surface in a case in which the treatment tool is pulled out, the treatment tool can be prevented from being caught on the guide surface.
(9) The endoscope according to any one of (1) to (8),
   in which the branching member and the introduction pipe line are integrally formed.
   According to (9), the manufacturing cost can be reduced. The size can be easily reduced.
(10) The endoscope according to any one of (1) to (9),
   in which the branching member is made of a resin.
   According to (10), the manufacturing cost can be reduced. In addition, the size can be easily reduced.

### Explanation of References

1A: insertion part
1a: soft portion
1b: bendable portion
1c: distal end portion
1g: insertion part holding portion
5d: holding portion
1m: locking projection
1t, 62e: edge
1: endoscope
2A: cable
2b, 2c: connector portion
2d: restricting portion
2: operating device
3A: first mounting portion
3Aa, 4Aa: button notch
3Ab, 4Ab: nozzle notch
3Ac, 4Ac: wall portion
3Ad, 4Ad: engaging groove
3B: second mounting portion
3C: third mounting portion
3Ca, 4Ca: pipe line notch
3Cb, 4Cb: rib
3d: attachment protrusion
3dg: fixing groove
3f: engaging portion
3i, 3j: pipe line protrusion
3k: rotation support portion
3m, 4m: holding end portion
3p, 4p: fitting hole
3: first case member
53: body part
4C: third mounting portion facing portion
4fh: engaging hole
4f: portion to be engaged
4: second case member
4k: rotation support portion
5b: nozzle hole
5: holding member
6a: operation lever portion
6b: pulley
6c: rotation support shaft
6d: finger rest portion
11a, 12a: brim portion
6p: guide member
6w: wire
6: bending operation portion
7: circuit board
8a: plane portion
8b: fitting end portion
8c: support hook
8d, 8e: side piece portion
8: support member
9D: lower-side surface
9L: left-side surface
9R: right-side surface
9U: upper-side surface
9b: cover member
9h: side surface opening
9Rr: rear-side surface
9Fr: front-side surface
9: housing
10: suction unit
11b: button body
11c, 11j, 61: connection portion
11s: operation element
11v: opening and closing valve
11: suction button
12a: brim portion
12b: nozzle body
12c: cap member
12d: restricting protrusion
12e: nozzle distal end
12f: locking projection
12g: projection to be locked
12h: arm portion
12i: engaging protrusion
12: suction nozzle
13a: one end
13c: the other end
13: connecting pipe
18: wire unit
20a: proximal end portion
20: insertion pipe line
21e, 22t: edge surface
21: first part
22e: end edge
22i: inner peripheral surface
22: second part
23t: cut surface
24: opening
25: annular distal end portion
30: suction pipe line
31: upper end part
32: lower end part
40A: treatment tool introduction opening
40e: branch position
40: introduction pipe line
41: flange portion
42: groove portion
50: branching member
51: fitting opening
52a: large-diameter stepped portion
52e: stepped surface
54e: contact surface
54: insertion pipe line side opening
60: connecting member
62c: guide surface
62: guide portion
90: treatment tool
91: case
93: suction device
94: suction pipe
100: endoscope system

## Claims

1. An endoscope comprising:
an insertion pipe line through which a treatment tool is inserted;
a suction pipe line;
an introduction pipe line into which the treatment tool is introduced; and
a branching member that branches the insertion pipe line into the introduction pipe line and the suction pipe line,
wherein a proximal end portion of the insertion pipe line is inserted into the branching member,
the proximal end portion includes a first part and a second part that is provided at a proximal end side with respect to the first part, and
an opening facing an introduction pipe line side is formed in an outer peripheral surface of the second part.

2. The endoscope according to claim 1,
wherein an inner peripheral surface of the second part intersects an extension line of an axis of the introduction pipe line.

3. The endoscope according to claim 2,
wherein a position of an end edge of the second part in an axial direction of the insertion pipe line is located at a suction pipe line side with respect to an intersection position between the extension line of the introduction pipe line and the inner peripheral surface.

4. The endoscope according to any one of claims 1 to 3,
wherein the branching member includes a contact surface that comes into contact with an edge surface at a second part side of the first part.

5. The endoscope according to claim 4,
wherein the contact surface is a surface perpendicular to an axial direction of the insertion pipe line.

6. The endoscope according to any one of claims 1 to 5,
wherein the branching member includes a body part that includes the introduction pipe line and an opening through which the insertion pipe line is inserted, and a connecting member that connects the body part and the suction pipe line to each other.

7. The endoscope according to claim 6,
wherein the connecting member includes a connection portion to which the suction pipe line is connected and a guide portion that guides a movement of the treatment tool inserted into the introduction pipe line.

8. The endoscope according to claim 7,
wherein the guide portion has a guide surface along the introduction pipe line, and
an edge of the guide surface is rounded.

9. The endoscope according to any one of claims 1 to 8,
wherein the branching member and the introduction pipe line are integrally formed.

10. The endoscope according to any one of claims 1 to 9,
wherein the branching member is made of a resin.
